(19) Europäisches Patentamt
European Patent Office
Office européen des brevets

(11) EP 3 949 847 A1

(12) **EUROPEAN PATENT APPLICATION**
published in accordance with Art. 153(4) EPC

(43) Date of publication:
**09.02.2022 Bulletin 2022/06**

(21) Application number: 20782333.7

(22) Date of filing: 30.03.2020

(51) International Patent Classification (IPC):
**A61B 5/0408** (2006.01) **A41B 9/06** (2006.01)
**A41D 13/12** (2006.01) **A41D 31/04** (2019.01)

(52) Cooperative Patent Classification (CPC):
**A41B 9/06; A41D 13/12; A41D 31/04; A61B 5/25;
A61B 5/291**

(86) International application number:
**PCT/JP2020/014441**

(87) International publication number:
**WO 2020/203944 (08.10.2020 Gazette 2020/41)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **29.03.2019 JP 2019067965**

(71) Applicants:
• **TOYOBO CO., LTD.**
  **Osaka-shi**
  **Osaka 530-8230 (JP)**
• **TOYOBO STC CO., LTD.**
  **Osaka-shi, Osaka 530-0004 (JP)**

(72) Inventors:
• **SHIMIZU, Yusuke**
  **Otsu-shi, Shiga 520-0292 (JP)**
• **KWON, Euichul**
  **Otsu-shi, Shiga 520-0292 (JP)**
• **MORIMOTO, Shota**
  **Otsu-shi, Shiga 520-0292 (JP)**
• **KOMATSU, Yoko**
  **Otsu-shi, Shiga 520-0292 (JP)**
• **MARUYAMA, Hiroshi**
  **Osaka-shi, Osaka 530-0004 (JP)**

(74) Representative: **Müller-Boré & Partner
Patentanwälte PartG mbB
Friedenheimer Brücke 21
80639 München (DE)**

(54) **GARMENT**

(57) The purpose of the present invention is to provide a biological information measurement garment that reduces the wear discomfort. The garment includes a clothing fabric; and an electrode formed on a skin-side surface of the clothing fabric, the clothing fabric having, in the skin-side surface thereof, an area of 30 cm$^2$ or more that is not covered with the electrode and is exposed, the electrode including a conductive member, and a heat capacity A (J/K) of the conductive member and a heat capacity B (J/K) of the clothing fabric satisfying a following formula (1).

$$-12\,(\text{J/K}) \le A - B \le 60\,(\text{J/K})\ (1)$$

FIG.1A

FIG.1B

**Description**

TECHNICAL FIELD

The present invention relates to a garment including a clothing fabric and an electrode formed on a skin-side surface of the clothing fabric. In detail, the present invention relates to a garment on which a biological information measuring electrode for detecting biological information of a wearer is formed. Specifically, the present invention relates to a biological information measuring garment having an electrode in direct contact with skin of a wearer, or an electrode to be a detection terminal of a sensor capable of proximally and contactlessly acquiring biological information.

BACKGROUND ART

In recent years, wearable biological information measurement apparatuses (sensing wear) are drawing attention in a health monitoring field, a medical field, an education field, and a rehabilitation field. The wearable biological information measurement apparatuses are apparatuses that include a biological information measurement apparatus provided on, for example, a belt or a strap, and that enables simple measurement of biological information, such as an electrocardiogram, of a person who wears it. As a biological information measurement apparatus, for example, an apparatus, in which a biological information measurement electrode in contact with skin of a wearer is formed, is known.

In cases of a garment-type wearable biological information measurement apparatus, an electrode is provided on a body clothing fabric formed of, for example, a woven fabric or a knitted fabric, making it possible to simply measure biological information, such as heartbeat variability, in various daily situations of a person who wears the garment and lives daily life.

Various garment-type wearable biological information measurement apparatuses have been known so far, and the inventors of the present invention have proposed, for example, in Patent Document 1, a sensing wear that determines a measurement position enabling the most stable measurement of biological information and to which a highly closely contactable flexible electrode is attached.

RELATED ART DOCUMENT

PATENT DOCUMENTS

Patent Document 1: JP-A-2017-29692

SUMMARY OF THE INVENTION

PROBLEM TO BE SOLVED BY THE INVENTION

Various garment-type wearable biological information measurement apparatuses have been known so far, but wearers who wears the garment have sometimes felt discomfort due to cool feeling caused by the electrode or the like provided on the skin-side surface of the garment. However, reduction of such wear discomfort has not been attempted so far. The present invention has been made focusing on the above problem, and an object of the present invention is to provide a biological information measurement garment that reduces the wear discomfort.

SOLUTIONS TO THE PROBLEMS

The garment according to the embodiment of the present invention that has solved the problem has the following configurations.

[1] A garment including:

a clothing fabric; and
an electrode formed on a skin-side surface of the clothing fabric,
the clothing fabric having, in the skin-side surface thereof, an area of 30 cm$^2$ or more that is not covered with the electrode and is exposed,
the electrode including a conductive member, and
a heat capacity A (J/K) of the conductive member and a heat capacity B (J/K) of the clothing fabric satisfying a following formula (1).

$$-12\,(\mathrm{J/K}) \le A - B \le 60\,(\mathrm{J/K})\ (1)$$

[2] The garment according to above [1], wherein the clothing fabric has a heat capacity B (J/K) of 230 (J/K) or more and 300 (J/K) or less.

[3] The garment according to above [1] or [2], wherein the conductive member has a heat capacity A (J/K) of 250 (J/K) or more and 350 (J/K) or less.

[4] The garment according to any one of above [1] to [3], wherein the clothing fabric has a basis weight of 230 g/m$^2$ or less.

[5] The garment according to any one of above [1] to [4], wherein the clothing fabric includes elastic yarns and non-elastic yarns.

[6] The garment according to above [5], wherein each of the non-elastic yarns includes a polyethylene multifilament yarn.

[7] The garment according to [6], having a blending proportion of the polyethylene multifilament yarn in 100 mass% of the clothing fabric of 2 mass% or more and 18 mass% or less.

[8] The garment according to any one of [1] to [7], wherein the conductive member is a conductive layer and contains 20 mass% or more and 98 mass% or less of a conductive filler.

[9] The garment according to any one of [1] to [8], wherein the conductive member is formed of a conductive fabric, and when the conductive fabric has a load of 14.7 N applied thereto in a body length direction or a body width direction, the conductive fabric has an extension percentage of 3% or more and 60% or less in at least one of the directions.

[10] The garment according to any one of [1] to [9], being an underwear top.

[11] The garment according to any one of [1] to [9], being an underwear bottom.

[12] The garment according to any one of [1] to [9], being a band-shaped fabric.

EFFECTS OF THE INVENTION

According to the present invention, the configurations described above enable provision of biological information measurement garment that reduces wear discomfort.

BRIEF DESCRIPTION OF THE DRAWINGS

Fig. 1A is a front view of a tab-attached T-shirt. Fig. 1B is a back view of the tab-attached T-shirt.

Fig. 2 is a perspective view of an electrode supporting portion with a double-open bag structure (or covered binding structure).

MODE FOR CARRYING OUT THE INVENTION

A garment according to the present invention includes a clothing fabric and an electrode formed on a skin-side surface of the clothing fabric, the clothing fabric having, in the skin-side surface thereof, an area of 30 cm$^2$ or more that is not covered with the electrode and is exposed, the electrode including a conductive member, and a heat capacity A (J/K) of the conductive member and a heat capacity B (J/K) of the clothing fabric satisfying a following formula (1).

$$-12\,(\mathrm{J/K}) \le A - B \le 60\,(\mathrm{J/K}) \qquad (1)$$

The configurations described above enables the garment to reduce the difference in the cool feeling between the clothing fabric and the electrode and therefore to reduce wear discomfort. In detail, a study of the inventors of the present invention has made it clear that the electrode that has an excessively higher cool feeling than the cool feeling of the clothing fabric generates wear discomfort, while the clothing fabric that has an excessively higher cool feeling than the cool feeling of the electrode also generates discomfort accompanying the difference in the cool feeling between both the electrode and the clothing fabric. Therefore, the inventors of the present invention have conducted an earnest study and, as a result, found that setting the heat capacity A (J/K) of the conductive member and the heat capacity B (J/K) of the clothing fabric so as to satisfy the formula (1) reduces the difference in the cool feeling between the clothing fabric and the electrode and enables the garment to reduce wear discomfort. Hereinafter, each of the configurations is described in detail.

The garment includes a clothing fabric and an electrode formed on a skin-side surface of the clothing fabric. Direct contact of an electrode surface of the electrode with skin of a wearer enables measurement of electrical signals from a body and thus enables measurement of biological information. As the biological information, information of a body, such as an electrocardiogram, a heart rate, a pulse rate, a breathing rate, blood pressure, body temperature, myopotential, and sweating, can be obtained by an electronic unit calculating and processing the electrical signals acquired through the electrode.

As the electrode, an electrode capable of measuring an electrocardiogram is preferable. The electrocardiogram means information obtained by detecting electrical changes caused by cardiac movement via an electrode on a surface of a living body and recording the changes as a waveform. The electrocardiogram is generally recorded as a waveform formed by plotting the potential difference, with the time put on the horizontal axis and the potential difference on the vertical axis. The waveform that appears every heartbeat on the electrocardiogram is mainly formed of representative five waves, i.e., a P wave, a Q wave, an R wave, an S wave, and a T wave, and there is also a U wave. A part of the start of a Q wave to the end of an S wave is sometimes called a QRS wave. An electrode capable of detecting at least the R wave among these waves is preferable. The R wave represents excitement of both the left and right ventricles and is a wave with the largest potential difference. Providing the electrode capable of detecting the R wave also enables measurement of a heart rate. That is, the time between the top of an R wave and the top of the next R wave is generally called an R-R interval (second), and the heart rate per minute can be calculated on the basis of the following formula. In the present specification, the QRS wave is to be included in the R wave unless otherwise noted. Specific configurations of the electrode and the conductive member are described later in detail.

$$\text{Heart rate (times/min)} = 60/\text{R-R interval}$$

A heat capacity A (J/K) of the conductive member and a heat capacity B (J/K) of the clothing fabric satisfy a following formula (1).

$$-12 \text{ (J/K)} \leq A - B \leq 60 \text{ (J/K)} \text{ (1)}$$

When the difference value between the heat capacity A (J/K) of the conductive member and the heat capacity B (J/K) of the clothing fabric is 60 (J/K) or less, the clothing fabric has an increased cool feeling and thus enables the cool feeling of the conductive member to be less likely to be sensed. For this reason, the difference value is preferably 50 (J/K) or less, more preferably 35 (J/K) or less, further preferably 25 (J/K) or less, further more preferably 15 (J/K) or less. On the other hand, when the difference value is -12 (J/K) or more, the difference in the cool feeling between the clothing fabric and the conductive member that accompanies an excessively high cool feeling of the clothing fabric can easily be reduced. For this reason, the difference value is preferably -10 (J/K) or more, more preferably -5 (J/K) or more, further preferably more than 0 (J/K). The heat capacity can be calculated together with specific heat by, for example, temperature-modulated differential scanning calorimetry (temperature-modulated DSC) described later in EXAMPLES. Calculation formulae of the heat capacity and the specific heat are the following formulae (2) and (3).

$$Cp = Kcp \times (Qamp/Tamp) \times (A/2\pi) \text{ (2)}$$

(In the formula, Cp represents the specific heat, Kcp represents a calibration coefficient of the specific heat, Qamp represents heat-flow amplitude (°C), Tamp represents heating amplitude (°C), and A represents a modulation period (second)).

$$C = m \times Cp \text{ (3)}$$

(In the formula, C represents the heat capacity (J/K), m represents mass (g), and Cp represents the specific heat (J/g·K).

The clothing fabric preferably has a heat capacity B (J/K) of 230 (J/K) or more and 300 (J/K) or less. The clothing fabric having a heat capacity B (J/K) of 230 (J/K) or more has an increased cool feeling and enables the cool feeling of the conductive member to be less likely to be sensed. The clothing fabric has a heat capacity B (J/K) of more preferably 235 (J/K) or more, further preferably 240 (J/K) or more, further more preferably 250 (J/K) or more. On the other hand, the clothing fabric having a heat capacity B (J/K) of 300 (J/K) or less can facilitate reduction of the difference in the cool feeling between the clothing fabric and the conductive member, the difference accompanying an excessively high cool feeling of the clothing fabric. For this reason, the clothing fabric has a heat capacity B (J/K) of more preferably 290 (J/K) or less, further preferably 280 (J/K) or less.

The conductive member preferably has a heat capacity A (J/K) of 250 (J/K) or more and 350 (J/K) or less. The conductive member having a heat capacity A (J/K) of 250 (J/K) or more can facilitate reduction of the difference in the cool feeling, the difference accompanying an excessively higher cool feeling of the clothing fabric than the cool feeling of the electrode. For this reason, the conductive member has a heat capacity A (J/K) of more preferably 260 (J/K) or more, further preferably 270 (J/K) or more, further more preferably 280 (J/K) or more. On the other hand, the conductive member having a heat capacity A (J/K) of 350 (J/K) or less can facilitate a reduced cool feeling. For this reason, the conductive member has a heat capacity A (J/K) of more preferably 320 (J/K) or less, further preferably 300 (J/K) or less.

The clothing fabric preferably has a basis weight of 230 g/m$^2$ or less. The clothing fabric having a basis weight of 230 g/m$^2$ or less can easily have a reduced weight. The clothing fabric has a basis weight of more preferably 220 g/m$^2$ or less, further preferably 200 g/m$^2$ or less. On the other hand, the clothing fabric having a basis weight of 150 g/m$^2$ or more can easily improve the strength. For this reason, the clothing fabric has a basis weight of more preferably 170 g/m$^2$ or more, further preferably 180 g/m$^2$ or more.

The clothing fabric has, in the skin-side surface thereof, an area of 30 cm$^2$ or more that is not covered with the electrode and is exposed. The exposure of the prescribed area of the clothing fabric on a skin-side surface of the garment enables the difference in the cool feeling between the clothing fabric and the conductive member to be less likely to be sensed. The clothing fabric has, in the skin-side surface thereof, an exposure area of preferably 50 cm$^2$ or more, more preferably 100 cm$^2$ or more, further preferably 200 cm$^2$ or more, further more preferably 400 cm$^2$ or more. On the other hand, the upper limit of the area on the skin-side surface of the clothing fabric may be, for example, 4000 cm$^2$ or less, 2000 cm$^2$ or less, 1000 cm$^2$ or less.

The garment preferably has, in the skin-side surface thereof, an area proportion of the skin-side surface of the clothing fabric of 20 area% or more. This configuration enables the difference in the cool feeling between the clothing fabric and the conductive member to be less likely to be sensed. The garment has an area proportion of the skin-side surface of the clothing fabric of more preferably 50 area% or more, further preferably 80 area% or more, further more preferably 95 area% or more, most preferably 100 area%.

The clothing fabric preferably includes elastic yarns and non-elastic yarns. The non-elastic yarns have no rubber elasticity. That is, the non-elastic yarns have lower stretchability than that of the elastic yarns and can thus easily prevent excessive stretch of the clothing fabric. As the non-elastic yarns, both filament yarns and spun yarns can be used. Specific examples of the non-elastic yarns include yarns including: multifilaments of synthetic fibers represented by polyethylene terephthalate, polytrimethylene terephthalate, nylon 6, nylon 66, an aramid, acrylic, an acrylate, polyethylene, polypropylene, a polyacrylate, and polybenzazole; chemical fibers (semi-synthetic fibers) represented by rayon, acetate, Lyocell, and cupra; natural fibers represented by cotton, wool, and silk; and carbon fibers. Only a single type of non-elastic yarns may be used, or two or more types of non-elastic yarns may be used. The non-elastic yarns may be either filament yarns or spun yarns, but are preferably filament yarns. As the filament yarns, multifilament yarns are preferable, and at least a single type of yarns selected from the group consisting of polyethylene multifilament yarns, polyethylene terephthalate multifilament yarns, and nylon 6 multifilament yarns is more preferable. This configuration can facilitate control of the heat capacity of the clothing fabric to about 230 to 300 (J/K).

Each of the non-elastic yarns preferably includes a polyethylene multifilament yarn. That is, the non-elastic yarn preferably includes at least a polyethylene multifilament yarn, and the non-elastic yarn including a polyethylene multifilament yarn can facilitate increase of the heat capacity of the clothing fabric. As the polyethylene multifilament yarn, a high molecular weight polyethylene multifilament yarn is preferable, and an ultra-high molecular weight polyethylene is more preferable. Normal polyethylene has a viscosity-average molecular weight of about 20000 to 300000, while high molecular weight polyethylene means polyethylene having a viscosity-average molecular weight of 500000 or more and less than 1000000, and ultra-high molecular weight polyethylene means polyethylene having a viscosity-average molecular weight of 1000000 or more.

The clothing fabric (100 mass%) preferably has a blending proportion of the polyethylene multifilament yarn of 2 mass% or more and 18 mass% or less. The clothing fabric having a blending proportion of the polyethylene multifilament yarn of 2 mass% or more can facilitate an increased cool feeling. For this reason, the clothing fabric has a blending proportion of the polyethylene multifilament yarn of preferably 5 mass% or more, more preferably 8 mass% or more. On the other hand, the clothing fabric having a blending proportion of the polyethylene multifilament yarn of 18 mass% or less facilitates an increased cool feeling and thus enables the cool feeling of the conductive member to be less likely to be sensed. For this reason, the clothing fabric has a blending proportion of the polyethylene multifilament yarn of preferably 15 mass% or less, more preferably 12 mass% or less.

The non-elastic yarns as a whole preferably have a specific heat of 0.9 (J/g·K) or more and 2.0 (J/g·K) or less. The non-elastic yarns as a whole having a specific heat of 0.9 (J/g·K) or more can facilitate increase of the heat capacity of the clothing fabric. On the other hand, the non-elastic yarns as a whole having a specific heat of 2.0 (J/g·K) or less can facilitate reduction of the heat capacity of the clothing fabric. The non-elastic yarns as a whole have a specific heat of more preferably 1.0 (J/g·K) or more and 1.6 (J/g·K) or less, further preferably 1.1 (J/g·K) or more and 1.4 (J/g·K) or less. The specific heat of the non-elastic yarns as a whole means, in the case of using a single type of non-elastic yarns, the specific heat of the non-elastic yarns themselves, and means, in the case of using two or more types of non-elastic yarns, an amount of heat J necessary for increasing the temperature by 1 K per 1 g of all the non-elastic yarns. The specific heat can be measured by the method described later in EXAMPLES.

The non-elastic yarns as a whole preferably have a heat capacity (J/K) of 220 (J/K) or more and 290 (J/K) or less. The non-elastic yarns as a whole having a heat capacity (J/K) of 220 (J/K) or more increase the cool feeling of the clothing fabric and enable the cool feeling of the conductive member to be less likely to be sensed. The non-elastic yarns as whole have a heat capacity (J/K) of more preferably 230 (J/K) or more, further preferably 235 (J/K) or more, further more preferably 240 (J/K) or more. On the other hand, the non-elastic yarns as a whole having a heat capacity (J/K) of 290 (J/K) or less can facilitate reduction of the difference in the cool feeling between the clothing fabric and the conductive member, the difference accompanying an excessively high cool feeling of the clothing fabric. For this reason, the non-elastic yarns as a whole have a heat capacity (J/K) of more preferably 280 (J/K) or less, further preferably 270 (J/K) or less. The heat capacity of the non-elastic yarns as a whole means, in the case of using a single type of non-elastic yarns, the heat capacity of the non-elastic yarns themselves, and means, in the case of using two or more types of non-elastic yarns, an amount of heat J necessary for increasing the temperature of all the non-elastic yarns by 1 K.

Each of the non-elastic yarns preferably has a single yarn fineness of 0.1 dtex or more and 3.0 dtex or less. The non-elastic yarn having a single yarn fineness of 0.1 dtex or more can facilitate increase of the cool feeling of the clothing fabric. For this reason, the non-elastic yarn has a single yarn fineness of preferably 0.5 dtex or more, more preferably 0.8 dtex or more. On the other hand, the non-elastic yarn having a single yarn fineness of 3.0 dtex or less can facilitate reduction of the cool feeling of the clothing fabric. For this reason, the non-elastic yarn has a single yarn fineness of more preferably 2.8 dtex or less, further preferably 2.5 dtex or less.

The single yarn fineness can be obtained, for example, by measuring the fineness based on corrected mass on the basis of 8.3.1 Method A of JIS L 1013 (2010), defining the resulting fineness as total fineness, and dividing the total fineness by the number of single fibers.

Each of the non-elastic yarns preferably has a total fineness of 10 dtex or more and 250 dtex or less. The non-elastic yarn having a total fineness of 10 dtex or more can facilitate increase of the cool feeling of the clothing fabric. For this reason, the non-elastic yarn has a total fineness of more preferably 30 dtex or more, further preferably 40 dtex or more. On the other hand, the non-elastic yarn having a total fineness of 250 dtex or less can facilitate reduction of the cool feeling of the clothing fabric. For this reason, the non-elastic yarn has a total fineness of more preferably 150 dtex or less, further preferably 100 dtex or less, further more preferably 80 dtex or less, particularly preferably 70 dtex or less.

The clothing fabric (100 mass%) preferably has a blending proportion of all the non-elastic yarns of 40 mass% or more and 90 mass% or less. The clothing fabric having a blending proportion of the non-elastic yarns of 40 mass% or more makes the heat capacity thereof close to the heat capacity of the conductive layer and can thus facilitate reduction of the difference in the cool feeling between the clothing fabric and the conductive layer. The clothing fabric has a blending proportion of the non-elastic yarns of more preferably 50 mass% or more, further preferably 60 mass% or more. On the other hand, setting the blending proportion of all the non-elastic yarns to 90 mass% or less makes the heat capacity of the clothing fabric close to the heat capacity of the conductive layer and can thus facilitate reduction of the difference in the cool feeling between the clothing fabric and the conductive layer. For this reason, the clothing fabric has a blending proportion of the non-elastic yarns of more preferably 85 mass% or less, further preferably 80 mass% or less.

The elastic yarns are yarns having rubber elasticity. The clothing fabric including the elastic yarns improves the stretchability and can thus easily reduce compression of garment worn. As the elastic yarns, both monofilaments and multifilaments can be used. Specific examples of the elastic yarns include polyurethane elastic yarns, polyester-based elastic yarns, polyolefin-based elastic yarns, natural rubber yarns, synthetic rubber yarns, and yarns made of stretchable composite fibers. Only a single type of elastic yarns may be used, or two or more types of elastic yarns may be used. Among these types of elastic yarns, polyurethane elastic yarns (spandex yarns) have excellent elasticity, an excellent heat-setting property, excellent chemical resistance, and the like, and are thus preferable. As the polyurethane elastic yarns, for example, fusion-type polyurethane elastic yarns, bonding polyurethane elastic yarns, and the like can be used.

Each of the elastic yarns preferably has a total fineness of 10 dtex or more and 180 dtex or less. The elastic yarn having a total fineness of 10 dtex or more can easily improve the stretchability of the clothing fabric. For this reason, the elastic yarn has a total fineness of more preferably 20 dtex or more, further preferably 30 dtex or more, further more preferably 40 dtex or more. On the other hand, the elastic yarn having a total fineness of 180 dtex or less enables easy reduction of the weight of the clothing fabric. For this reason, the elastic yarn has a total fineness of more preferably 100 dtex or less, further preferably 90 dtex or less, further more preferably 80 dtex or less.

The clothing fabric (100 mass%) preferably has a blending proportion of the elastic yarns of 10 mass% or more and 60 mass% or less. The clothing fabric having a blending proportion of the elastic yarns of 10 mass% or more can easily improve the stretchability. The clothing fabric has a blending proportion of the elastic yarns of more preferably 15 mass% or more, further preferably 20 mass% or more. On the other hand, setting the blending proportion of the elastic yarns to 60 mass% or less enables knitting and a dyeing process to be easily performed and thus improves the productivity. Further, the clothing fabric having such a blending proportion gives a good fit and enables dimensional changes to be less likely to be generated. The clothing fabric has a blending proportion of the elastic yarns of more preferably 50 mass% or less, further preferably 40 mass% or less, further more preferably 35 mass% or less.

The elastic yarns as a whole preferably have a specific heat of 1.2 (J/g·K) or more and 2.0 (J/g·K) or less. The elastic yarns as a whole having a specific heat of 1.2 (J/g·K) or more can facilitate increase of the heat capacity of the clothing fabric. On the other hand, the non-elastic yarns as a whole having a specific heat of 2.0 (J/g·K) or less can facilitate reduction of the heat capacity of the clothing fabric. The elastic yarns as a whole have a specific heat of more preferably 1.4 (J/g·K) or more and 1.9 (J/g·K) or less, further preferably 1.6 (J/g·K) or more and 1.85 (J/g·K) or less. The specific heat of the elastic yarns as a whole means, in the case of using a single type of elastic yarns, the specific heat of the elastic yarns themselves, and means, in the case of using two or more types of elastic yarns, an amount of heat J necessary for increasing the temperature by 1 K per 1 g of all the elastic yarns. The specific heat can be measured by the method described later in EXAMPLES.

The elastic yarns as a whole preferably have a heat capacity (J/K) of 10 (J/K) or more and 100 (J/K) or less. The elastic yarns as a whole having a heat capacity (J/K) of 10 (J/K) or more increase the cool feeling of the clothing fabric and enable the cool feeling of the conductive member to be less likely to be sensed. The elastic yarns as a whole have a heat capacity (J/K) of more preferably 20 (J/K) or more, further preferably 30 (J/K) or more. On the other hand, the elastic yarns as a whole having a heat capacity (J/K) of 100 (J/K) or less can facilitate reduction of the difference in the cool feeling between the clothing fabric and the conductive member, the difference accompanying an excessively high cool feeling of the clothing fabric. For this reason, the elastic yarns as a whole have a heat capacity (J/K) of more preferably 80 (J/K) or less, further preferably 60 (J/K) or less. The heat capacity of the elastic yarns as a whole means, in the case of using a single type of elastic yarns, the heat capacity of the elastic yarns themselves, and means, in the case of using two or more types of elastic yarns, an amount of heat J necessary for increasing the temperature of all the elastic yarns by 1 K.

The clothing fabric has a blending ratio of the polyethylene multifilament yarns to 100 parts by mass of the elastic yarns of preferably 15 parts by mass or more and 75 parts by mass or less, more preferably 20 parts by mass or more and 70 parts by mass or less, further preferably 25 parts by mass or more and 60 parts by mass or less. This configuration makes the clothing fabric facilitate an increase of the cool feeling attributing to the polyethylene multifilament yarns and an improvement of the stretchability attributing to the elastic yarns.

The clothing fabric preferably includes the elastic yarns and the non-elastic yarns. Specific examples of the clothing fabric include a woven or knitted fabric obtained by interweaving or interknitting the elastic yarns and the non-elastic yarns. The woven or knitted fabric means a woven fabric or a knitted fabric.

The knitted fabric may be a weft knitted fabric or a warp knitted fabric, but is preferably a warp knitted fabric. The weft knitted fabric includes a circular knitted fabric.

Examples of the weft knitted fabric (circular knitted fabric) include fabrics having stitch structures such as plain stitch (jersey stitch), bare jersey stitch, welt jersey stitch, rib stitch, pearl stitch, half tubular stitch, interlock stitch, tuck stitch, float stich, half cardigan stitch, lace stitch, and plating stitch. The weft knitted fabric may be a single knit or a double knit.

Examples of the warp knitted fabric include fabrics having stitch structures such as single denbigh stitch, open-loop denbigh stitch, single atlas stitch, double cord stitch, half stitch, half base stitch, satin stitch, tricot stitch, half tricot stitch, raschel stitch, and jacquard stitch. Among these warp knitted fabrics, a fabric having tricot stitch as a stitch structure is preferable.

Examples of the woven fabric include woven fabrics formed by plain weave, twill weave, and satin weave. The woven fabric is not limited to a single woven fabric, but may be a multiple woven fabric such as a double woven fabric or a triple woven fabric.

Next, the electrode provided on the garment is described. The electrode includes a conductive member. Examples of the conductive member include a conductive layer and a conductive fabric (conductive structure).

The conductive layer is a layer that contacts with skin of a living body and thus detects electrical information of the living body, and transfers electrical signals. The conductive layer preferably includes resin and a conductive filler, more preferably includes a conductive filler and stretchable resin, further preferably includes a conductive filler and elastomer. The conductive layer can be formed using, for example, a composition (hereinafter, sometimes referred to as a conductive paste) obtained by dissolving or dispersing components in an organic solvent. The conductive layer is preferably sheet-shaped, more preferably a sheet-shaped product formed from a conductive composition including a conductive filler and stretchable resin.

The conductive layer preferably contains 20 mass% or more and 98 mass% or less of the conductive filler. The conductive layer having a content of the conductive filler of 98 mass% or less can facilitate reduction of the cool feeling of the electrode. For this reason, the conductive layer has a content of the conductive filler of more preferably 95 mass% or less, further preferably 90 mass% or less, further more preferably 88 mass% or less. On the other hand, the conductive layer having a content of the conductive filler of 20 mass% or more can facilitate increase of the cool feeling of the electrode while improving the conductivity. For this reason, the conductive layer has a content of the conductive filler of more preferably 30 mass% or more, further preferably 40 mass% or more, further more preferably 60 mass% or more, particularly preferably 70 mass% or more. The content can also be said as content (mass%) of the conductive filler with respect to the total solid content of the conductive paste for forming the conductive layer. As the conductive layer, it is acceptable to use a plurality of conductive layers that are integrated by stacking or aligning two or more types of conductive layers, the two or more types of conductive layers being obtained by changing the type of the conductive filler, the addition amount of the conductive filler, and the like.

The conductive layer can be produced, for example, by performing printing with the conductive paste on a prescribed base material. As the conductive layer, a sheet can also be used that is obtained by coating a prescribed base material with the conductive paste and cutting out the resulting coating film into a prescribed shaped as necessary.

At least a part of the conductive layer is exposed on the skin-side surface of the garment, and the garment preferably has, in the skin-side surface thereof, a total exposure area of the conductive layer of 5 cm$^2$ or more and 100 cm$^2$ or less. The garment having a total exposure area of the conductive layer of 5 cm$^2$ or more can easily acquire biological information. The garment has a total exposure area of the conductive layer of more preferably 10 cm$^2$ or more, further preferably 15 cm$^2$ or more. On the other hand, the garment having a total exposure area of the conductive layer of 100 cm$^2$ or less can facilitate reduction of the cool feeling of the conductive layer. The garment has a total exposure area of the conductive layer of more preferably 60 cm$^2$ or less, further preferably 30 cm$^2$ or less.

As the conductive filler, it is possible to use, for example, a metal powder, metal nanoparticles, and a conductive material other than the metal powder. A single type of material, or two or more types of materials may be used as the conductive filler. Examples of the metal powder include noble metal powders such as a silver powder, a gold powder, a platinum powder, and a palladium powder; base metal powders such as a copper powder, a nickel powder, an aluminum powder, and a brass powder; a plated powder obtained by plating different types of particles made of inorganic substances such as a base metal and silica with a noble metal such as silver; and an alloyed base metal powder obtained by alloying a base metal and a noble metal such as silver. Among these materials, a silver powder and/or a copper is preferable and enables the conductive layer to exhibit high conductivity at low costs. The silver powder and/or the copper powder is preferably a main component of the metal powder used as the conductive filler, and the main component means a total of 50 mass% or more. Examples of the metal nanoparticles include particles having a particle size of several to several tens of nanometers among the metal powders described above.

Examples of the conductive material other than the metal powder include carbon-based materials such as graphite, carbon black, and carbon nanotube. The conductive material other than the metal power preferably has a mercapto group, an amino group, or a nitrile group on a surface thereof, or preferably has a surface thereof surface-treated with rubber containing a sulfide bond and/or a nitrile group. Generally, the conductive material other than the metal powder has, by itself, strong agglomeration force, and has bad dispersibility in resin when the conductive material other than the metal powder has a high aspect ratio. However, by having a mercapto group, an amino group, or a nitrile group on the surface, or by being surface-treated with rubber containing a sulfide bond and/or a nitrile group, the conductive material other than the metal powder increases the affinity for resin and is dispersed, enabling formation of an effective conductive network and thus realization of high conductivity. The conductive filler has a proportion of the conductive material other than the metal powder of preferably 20 vol% or less, more preferably 15 vol% or less, further preferably 10 vol% or less. The conductive filler having an excessively large content proportion of the conductive material other than the metal powder sometimes has difficulty being uniformly dispersed in resin, and the conductive materials other than the metal powder that are described above are generally expensive. Therefore, it is preferable to suppress the use amount of the conductive material other than the metal powder to the above range.

The stretchable resin preferably includes at least, for example, rubber containing a sulfur atom and/or rubber containing a nitrile group. The sulfur atom and the nitrile group have high affinity for the conductive filler (particularly, the metal powder), and the rubber is highly stretchable. Therefore, the load per unit width in 10% extension of the electrode and the wiring can be reduced and the generation of a crack or the like in extension can be avoided. In addition, such resin can retain uniform dispersion of the conductive filler even when the electrode and the wiring are extended, and can therefore reduce the variation of the electric resistance in 20% extension and enable the electrode and the wiring to exhibit excellent conductivity. Further, such resin enables the electrode and the wiring to exhibit excellent conductivity even when the thickness of the electrode and the wiring is reduced. Between two types of rubber, rubber containing a nitrile group is more preferable and can furthermore reduce the variation of the electric resistance in 20% extension.

As the rubber containing a sulfur atom, elastomer may be used besides the rubber containing a sulfur atom. The sulfur atom is contained in the form of a sulfide bond or a disulfide bond in the main chain of a polymer, a mercapto group in a side chain or at a terminal, or the like. Examples of the rubber containing a sulfur atom include polysulfide rubber, polyether rubber, polyacrylate rubber, and silicone rubber that contain a mercapto group, a sulfide bond, or a disulfide bond. Particularly, polysulfide rubber, polyether rubber, polyacrylate rubber, and silicone rubber that contain a mercapto group are preferable. As a commercially available product that can be used as the rubber containing a sulfur atom, a preferable example is liquid polysulfide rubber "THIOKOL (registered trademark) LP" manufactured by Toray Fine Chemicals Co., Ltd. The rubber containing a sulfur atom preferably has a content of the sulfur atom of 10 to 30 mass%. As the rubber containing no sulfur atom, it is also possible to use, for example, resin in which a sulfur-containing compound such as pentaerythritol tetrakis(S-mercaptobutyrate), trimethylolpropane tris(S-mercaptobutyrate), or mercapto group-containing silicone oil is blended.

As the rubber containing a nitrile group, elastomer may be used besides the rubber containing a nitrile group. Particularly, an acrylonitrile-butadiene copolymer rubber that is a copolymer of butadiene and acrylonitrile is a preferable example. As a commercially available product that can be used as the rubber containing a nitrile group, preferable examples are Nipol (registered trademark) 1042 and Nipol (registered trademark) DN003 that are manufactured by Zeon Corporation. The rubber containing a nitrile group has an amount of the nitrile group (particularly, the acrylonitrile-butadiene copolymer rubber has an amount of acrylonitrile) of preferably 18 to 50 mass%, more preferably 20 to 45 mass%, further preferably 28 to 41 mass%. Particularly, the acrylonitrile-butadiene copolymer rubber having an excessively large amount of bonded acrylonitrile increases the affinity for the conductive filler, particularly the metal powder, but inversely reduces the rubber elasticity contributing to the stretchability.

A single type of resin, or two or more types of resin may be used as the stretchable resin that forms the conductive layer. That is, the stretchable resin that forms the conductive layer is preferably formed of only the rubber containing a sulfur atom and the rubber containing a nitrile group, but may contain stretchable resin other than the rubber containing a sulfur atom and the rubber containing a nitrile group in a range not to impair, for example, conductivity, stretchability, coatability during forming the conductive layer. When including another type of stretchable resin, the entire resin has a total amount of the rubber containing a sulfur atom and the rubber containing a nitrile group of preferably 95 mass% or more, more preferably 98 mass% or more, further preferably 99 mass% or more. The conductive layer has a content of the stretchable resin (in other words, the conductive paste (total solid content) for forming the conductive layer has a content of the stretchable resin (solid content)) of preferably 2 mass% or more and 80 mass% or less, more preferably 5 mass% or more and 60 mass% or less, further preferably 12 mass% or more and 30 mass% or less. The conductive layer having a content of the stretchable resin of 2 mass% or more improves the stretchability, and the conductive layer having a content of the stretchable resin of 80 mass% or less can facilitate increase of the cool feeling of the electrode.

The conductive layer can be formed directly on the first insulating layer described later, using a composition (conductive paste) obtained by dissolving or dispersing the components described above in an organic solvent, or can be formed by forming a coating film in a desired pattern through coating or printing, and volatilizing the organic solvent included in the coating film and thus drying the coating film. The conductive layer may also be formed by applying the conductive paste to or performing printing with the conductive paste on a release sheet or the like to form a coating film, volatilizing the organic solvent included in the coating film and thus drying the coating film to form a sheet-shaped conductive layer in advance, and stacking the conductive layer in a desired pattern on the first insulating layer described later. The conductive paste may be prepared employing a conventionally known method for dispersing a powder substance in a liquid, and can be prepared by uniformly dispersing the conductive filler in the stretchable resin. The conductive paste may be prepared by mixing, for example, the metal powder, the metal nanoparticles, or the conductive material other than the metal powder with a resin solution, and then uniformly dispersing the contents by an ultrasonic method or with a mixer, a triple ball mill, or a ball mill. A plurality of these types of means can be used in combination. The method for applying the conductive paste or performing printing with the conductive paste is not particularly limited, and a printing method can be employed, such as coating, screen printing, litho offset printing, ink-jet printing, flexo printing, gravure printing, gravure offset printing, stamping, dispensing, or squeegee printing.

The conductive layer preferably has a basis weight of 300 g/m$^2$ or more and 500 g/m$^2$ or less. The conductive layer having a basis weight of 500 g/m$^2$ or less can facilitate a reduced cool feeling. The conductive layer has a basis weight of more preferably 450 g/m$^2$ or less, further preferably 420 g/m$^2$ or less. On the other hand, the conductive layer having a basis weight of 300 g/m$^2$ or more can facilitate an increased cool feeling. For this reason, the conductive layer has a basis weight of more preferably 350 g/m$^2$ or more, further preferably 370 g/m$^2$ or more.

The conductive layer has a dried film thickness of preferably 10 to 150 pm, more preferably 20 to 130 $\mu$m, further preferably 30 to 100 $\mu$m. The conductive layer having a dried film thickness of 10 $\mu$m or more makes the electrode and the wiring less likely to be deteriorated even subjected to repetitive stretch and contraction, and makes the conduction less likely to be inhibited or blocked. On the other hand, the conductive layer having a dried film thickness of 150 $\mu$m or less improves the stretchability and thus facilitate improvement of wear comfort.

Specific examples of the conductive fabric include a woven fabric, a knitted fabric, and a nonwoven fabric made of: conductive fibers such as a fiber obtained by covering a base fiber with a conductive polymer and a fiber having a surface thereof covered with a conductive metal such as silver, gold, copper, or nickel; or conductive yarns such as a conductive yarn made of a conductive fine metal wire and a conductive yarn obtained by mixed spinning of a conductive fine metal wire and a non-conductive fiber. The conductive fabric may include only a single type or two or more types of these conductive fibers and conductive yarns. A non-conductive fabric embroidered using conductive yarns may also be used as the conductive fabric.

When the conductive fabric has a load of 14.7 N applied thereto in a body length direction or a body width direction, the conductive fabric preferably has an extension percentage of 3% or more and 60% or less in at least one of the directions. The conductive fabric having an extension percentage of 3% or more allows the electrode to easily follow the movement of the clothing fabric of the garment and enables the electrode to easily avoid being peeled from the clothing fabric. For this reason, the conductive fabric has an extension percentage of preferably 3% or more, more preferably 5% or more, further preferably 10% or more. On the other hand, the conductive fabric having an extension percentage of 60% or less enables the electrode to avoid being excessively stretched and can thus easily improve the accuracy of biological information. For this reason, the conductive fabric has an extension percentage of preferably 60% or less, more preferably 55% or less, further preferably 50% or less. The conductive fabric satisfies the extension percentage within the above range preferably in the body length direction or the body width direction, and more preferably in both the body length direction and the body width direction.

The electrode preferably includes a first insulating layer formed on the skin-side surface of the clothing fabric, and the conductive layer formed on a skin-side surface of the first insulating layer. However, the electrode may be formed on the skin-side surface of the clothing fabric by forming the conductive layer directly on the skin-side surface of the clothing fabric.

The garment preferably includes, in addition to the electrode, wiring connecting the electrode to an electronic unit or the like having a function of calculating electrical signals acquired through the electrode. The wiring preferably includes the first insulating layer formed on the skin-side surface of the clothing fabric, the conductive layer formed on a skin-side surface of the first insulating layer, and a second insulating layer formed on a skin-side surface of the conductive layer. Hereinafter, the first insulating layer and the second insulating layer are specifically described.

(First insulating layer)

The first insulating layer not only acts as an insulating layer, but also acts as an adhesive layer for forming on the clothing fabric the conductive layer of the electrode and the wiring and acts as a waterproof layer that prevents water from reaching the conductive layer, the water coming from the side opposite from the side, on which the first insulating layer is stacked, of the clothing fabric of the garment worn (that is, the outer side of the garment). In addition, the first insulating layer provided on the garment side of the conductive layer can suppress stretch of the clothing fabric and thus prevent the conductive layer from being excessively extended. As a result, the first insulating layer can be prevented from generating a crack. Contrarily, although the conductive layer has good extensibility as described above, the conductive layer which is directly formed on the surface of the clothing fabric is excessively stretched following the stretch of the clothing fabric when the clothing fabric is a material having a good stretch property exceeding the extensibility of the conductive layer. The conductive layer is considered to generate a crack as a result of this phenomenon.

The first insulating layer may be formed of insulating resin, and the type of resin is not particularly limited. As the resin, it is possible to preferably use, for example, polyurethane-based resin, silicone-based resin, vinyl chloride-based resin, epoxy-based resin, and polyester elastomer. Among these types of resin, polyurethane-based resin is more preferable, and has further good adhesiveness to the conductive layer. A single type of resin, or two or more types of resin may be used as the resin forming the first insulating layer. The method for forming the first insulating layer is not particularly limited, and can be formed, for example, by dissolving or dispersing the insulating resin in a solvent (preferably water), applying the resulting solution to or performing printing with the resulting solution on release paper or a release film to form a coating film, and volatilizing the solvent included in the coating film and thus drying the coating film. A commercially available resin sheet or resin film can also be used.

The first insulating layer preferably has an average film thickness of 10 to 200 $\mu$m. The first insulating layer that is excessively thin sometimes has an insufficient insulating effect and an insufficient stretch preventing effect. Accordingly, the first insulating layer has an average film thickness of preferably 10 $\mu$m or more, more preferably 30 $\mu$m or more, further preferably 40 $\mu$m or more. However, the first insulating layer that is excessively thick sometimes impairs the stretchability of the electrode and the wiring. In addition, the first insulating layer that is excessively thick makes the electrode and the wiring excessively thick and may thus possibly deteriorate wear comfort. Accordingly, the first insulating layer has an average film thickness of preferably 200 $\mu$m or less, more preferably 180 $\mu$m or less, further preferably 150 $\mu$m or less.

(Second insulating layer)

The wiring preferably includes a second insulating layer formed on the conductive layer. The second insulating layer provided can prevent the conductive layer from contacting with, for example, water such as rain, snow, and sweat. Examples of resin forming the second insulating layer include the same types of resin as the above-described resin forming the first insulating layer, and preferably used resin is also the same. A single type of resin, or two or more types of resin may also be used as the resin forming the second insulating layer. The resin forming the second insulating layer may be the same as or different from the resin forming the first insulating layer, but is preferably the same. The use of the same resin can reduce damage on the conductive layer caused by bias in coverage of the conductive layer and in stress during stretch or contraction of the wiring. The second insulating layer can be formed by the same forming method as the first insulating layer. A commercially available resin sheet or resin film can also be used.

The second insulating layer preferably has an average film thickness of 10 to 200 $\mu$m. The second insulating layer that is excessively thin is easily deteriorated when repetitively stretched and contracted, and thus sometimes has an insufficient insulating effect. Accordingly, the second insulating layer has an average film thickness of preferably 10 $\mu$m or more, more preferably 30 $\mu$m or more, further preferably 40 $\mu$m or more. However, the second insulating layer that is excessively thick impairs the stretchability of the wiring, and makes the wiring excessively thick and may thus possibly deteriorate wear comfort. Accordingly, the second insulating layer has an average film thickness of preferably 200 $\mu$m or less, more preferably 180 $\mu$m or less, further preferably 150 $\mu$m or less.

The electrode and the wiring preferably have a load per unit width in 10% extension of 100 N/cm or less. When having a load per unit width in 10% extension of more than 100 N/cm, the electrode and the wiring have difficulty making the extension thereof follow the extension of the clothing fabric and thus sometimes impair wear comfort of the garment worn. Accordingly, the electrode and the wiring have a load per unit width in 10% extension of preferably 100 N/cm or less, more preferably 80 N/cm or less, further preferably 50 N/cm or less.

The electrode and the wiring preferably have a variation of electric resistance in 20% extension of 5 times or less. The electrode and the wiring that have a variation of electric resistance in 20% extension of more than 5 times remarkably decrease the conductivity. Accordingly, the electrode and the wiring have a variation of electric resistance in 20% extension of preferably 5 times or less, more preferably 4 times or less, further preferably 3 times or less.

The electrode and the wiring may be formed of different materials, but are preferably formed of the same material. When the electrode and the wiring are formed of the same material, the wiring is set to have a width of preferably 1 mm or more, more preferably 3 mm or more, further preferably 5 mm or more. The upper limit of the wiring width is not particularly limited, but is preferably set to, for example, 10 mm or less, more preferably 9 mm or less, further preferably 8 mm or less.

The electrode and the wiring are preferably formed directly on the clothing fabric forming the garment. The method for forming the electrode and the wiring on the clothing fabric is not particularly limited as long as it does not prevent establishment of the stretchability of the electrode and the wiring. From the viewpoints of a good fit of the garment worn and the following property of the garment in exercise and motion, for example, a known method can be employed, such as stacking with an adhesive or stacking by heat pressing. When the electrode and the wiring are formed by stacking with an adhesive or stacking by heat pressing, the clothing fabric preferably has no material attached thereto that impairs the adhesiveness, such as a silicon-based softer or a fluorine-based water repellent. The amount of the silicon-based softener, the fluorine-based water repellent, or the like attached to the clothing fabric can be adjusted by methods such as removing the silicon-based softener used for the elastic yarns or the like in scouring treatment of a step of dyeing the clothing fabric, or selecting the type of a processing agent used in finishing and setting the clothing fabric.

The electrode is preferably provided in a chest part or an abdominal part under the chest of the garment. The electrode provided in the chest part or the abdominal part under the chest of the garment enables accurate measurement of biological information. The electrode is more preferably provided, on the garment, in a region that contacts with skin between the upper end of the seventh rib and the lower end of the ninth rib of a wearer. The electrode is preferably provided, on the garment, in a ventral region of a wearer between lines that are parallel with left and right posterior axillary lines of the wearer and are drawn at positions 10 cm backward away from the posterior axillary lines of the wearer. The electrode is preferably provided circularly along the waistline of the wearer. The garment is provided with at least two electrodes, and the two electrodes are preferably provided in a chest part or an abdominal part under the chest of the garment, and the two electrodes are preferably provided in a ventral region of a wearer between lines that are parallel with left and right posterior axillary lines of the wearer and are drawn at positions 10 cm backward away from the posterior axillary lines of the wearer. This region is a site that is relatively sensitive to the cool feeling, and therefore the electrode that more easily gives a cool feeling than the garment easily makes a wearer have a feeling of a foreign body. However, the clothing fabric according to the present invention reduces the difference in the cool feeling between the electrode and the clothing fabric and therefore enables a wearer to comfortably wear the garment hardly having a feeling of a foreign body. When three or more electrodes are provided, the positions at which the third and following electrodes are provided are not particularly limited, and may be provided, for example, on the clothing fabric of a back body.

The electrode has an electric resistance value on an electrode surface of preferably 1000 $\Omega$/cm or less, more preferably 300 $\Omega$/cm or less, further preferably 200 $\Omega$/cm or less, particularly preferably 100 $\Omega$/cm or less. Particularly, the electrode having a sheet shaped-form can usually suppress the electric resistance on the electrode surface to 300 $\Omega$/cm or less.

The form of the electrode is preferably a sheet shape. The electrode formed in a sheet shape can have a wide electrode surface and can therefore secure the contact area with skin of a wearer. The sheet-shaped electrode preferably has good flexibility. The sheet-shaped electrode is also preferably stretchable. The size of the sheet-shaped electrode is not particularly limited as long as it can measure electrical signals from a body. The electrode has an area of the electrode surface of 5 to 100 cm$^2$ and preferably has an average thickness of 10 to 500 $\mu$m. The electrode has an area of the electrode surface of more preferably 10 cm$^2$ or more, further preferably 15 cm$^2$ or more. The electrode has an area of the electrode surface of more preferably 90 cm$^2$ or less, further preferably 80 cm$^2$ or less. The electrode that is excessively thin sometimes has insufficient conductivity. Accordingly, the electrode has an average thickness of preferably 10 $\mu$m or more, more preferably 30 $\mu$m or more, further preferably 50 $\mu$m or more. However, the electrode that is excessively thick sometimes gives a wearer a feeling of a foreign body and thus discomfort. Accordingly, the electrode has an average thickness of preferably 500 $\mu$m or less, more preferably 450 $\mu$m or less, further preferably 400 $\mu$m or less. The shape of the electrode is not particularly limited as long as it goes along the curve of a body corresponding to the position at which the electrode is disposed and easily allows the electrode to follow the movement of the body and closely contact with the body. Examples of the shape include a tetragon, a triangle, a five or more sided polygon, a circle, and an ellipse. When the electrode has a polygonal shape, the vertices of the polygon may be rounded not to allow a scratch on skin.

The wiring may be formed using a conductive fiber or a conductive yarn. As the conductive fiber or the conductive yarn, it is possible to use, for example, one obtained by plating a surface of an insulating fiber with a metal, one obtained by twisting a fine metal wire in a yarn, one obtained by impregnating a conductive polymer into between fibers such as microfibers, and a fine metal wire. The wiring preferably has an average thickness of 10 to 500 $\mu$m. The wiring having an excessively small thickness sometimes has insufficient conductivity. Accordingly, the electrode has an average thickness of preferably 10 $\mu$m or more, more preferably 30 $\mu$m or more, further preferably 50 $\mu$m or more. However, the wiring having an excessively large thickness sometimes gives a wearer a feeling of a foreign body and thus discomfort. Accordingly, the wiring has an average thickness of preferably 500 $\mu$m or less, more preferably 300 $\mu$m or less, further preferably 200 $\mu$m or less. The shape of the wiring is not particularly limited and may be a redundant geometrical pattern as well as a straight line and a curve. Examples of the redundant geometrical pattern include a zigzag shape, a continuous horseshoe shape, and a wave shape. The electrode in the redundant geometrical pattern can be formed using, for example, a metal foil.

The form of the garment is not particularly limited as long as it allows formation of the electrode, and is preferably underwear, a band-shaped fabric, or the like. In addition, the garment may be a fabric that covers at least any one of a breast, a hand, a leg, a foot, a neck, and a face.

As the underwear, an underwear top or an underwear bottom is preferable. Examples of the underwear top include a T-shirt, a polo shirt, a camisole, a brassiere, sports innerwear, a hospital gown, and pajamas. Examples of the underwear bottom include pants, sports innerwear, a hospital gown, and pajamas.

Examples of the band-shaped fabric include a belt, and specific examples thereof include a breast belt and an abdominal belt.

The garment preferably includes, on a surface opposite from the skin-side surface of the clothing fabric, a fastener used for connection to the electronic unit. The fastener is a so-called hook, and examples thereof include a stainless-steel hook. The conductive layer and the electronic unit can be electrically connected to each other via the fastener.

The electronic unit or the like is preferably detachable from the garment. Further, the electronic unit or the like preferably includes display means, storage means, communication means, a USB connector, and the like. The electronic unit or the like may also include, for example, a sensor capable of measuring environmental information such as atmospheric temperature, humidity, or atmospheric pressure, or a sensor capable of measuring positional information using a GPS.

Using the garment also enables application to a technique of grasping a human psychological or physiological state. For example, the degree of relaxation can be detected for mental training, sleepiness can be detected for preventing drowsy driving, or an electrocardiogram can be measured for diagnosis of depression, stress, or the like.

The present application claims priority based on Japanese Patent Application No. 2019-067965 filed on March 29, 2019. All the contents described in Japanese Patent Application No. 2019-067965 filed on March 29, 2019 are incorporated herein by reference.

EXAMPLES

Hereinafter, the present invention is more specifically described by way of examples. The present invention is not limited by the following examples, and can be modified and implemented within the scope of complying with the gist

EP 3 949 847 A1

of the descriptions above and below. Those all fall within the technical scope of the present invention.

Fineness: The single yarn fineness was obtained by measuring the fineness based on corrected mass on the basis of 8.3.1 Method A of JIS L 1013 (2010), defining the resulting fineness as total fineness, and dividing the total fineness by the number of single fibers.

Basis weight: In accordance with "Mass per unit area in standard state" specified in 8.3.2 of JIS L 1096 (2010), the basis weight was measured.

Specific heat and heat capacity: The specific heat and the heat capacity of the warp knitted fabrics and the conductive layer were measured by temperature-modulated differential scanning calorimetry (temperature-modulated DSC) under the following conditions. In detail, each of the warp knitted fabrics was cut into pieces and 5 mg of the fabric was collected. On the other hand, the conductive layer was cut in a several mm square, and 5 mg of the conductive layer was collected. Each of the obtained samples was packed in a container. Thereafter, calibration of the device was performed using sapphire as a standard material, and then the specific heat and the heat capacity of each of the samples were measured.

Measurement device: Q100 manufactured by TA instruments
Temperature range: 0°C to 50°C
Temperature rise rate: 2°C/min
Temperature modulation period: 60 seconds
Temperature modulation amplitude: 0.32°C

Example 1

High molecular weight polyethylene yarns (IZANAS SK60 55 T/43 f manufactured by TOYOBO CO., LTD.) nylon 6 yarns (44 T/40 f), and spandex yarns (78 T) at the mass ratio shown in Table 1 were knitted in tricot stitch into a warp knitted fabric. T means dtex and f means filaments. For example, 55T43f in the table means a multifilament yarn that is formed of 43 filaments and has a total fineness of 55 dtex.

Example 2

High molecular weight polyethylene yarns (55 T/43 f), nylon 6 yarns (44 T/40 f), and spandex yarns (78 T) at the mass ratio shown in Table 1 were knitted in tricot stitch into a warp knitted fabric.

Example 3

High molecular weight polyethylene yarns (55 T/43 f), polyethylene terephthalate yarns (56 T/24 f), and spandex yarns (78 T) at the mass ratio shown in Table 1 were knitted in tricot stitch into a warp knitted fabric.

Comparative Example 1

Nylon 6 yarns (44 T/40 f) and spandex yarns (78 T) at the mass ratio shown in Table 1 were knitted in tricot stitch into a warp knitted fabric.

Comparative Example 2

High molecular weight polyethylene yarns (55 T/43 f), polyethylene terephthalate yarns (56 T/24 f), and spandex yarns (78 T) at the mass ratio shown in Table 1 were knitted in tricot stitch into a warp knitted fabric.

The specific heat, the heat capacity, and the basis weight of the warp knitted fabrics were measured under the above conditions. Table 1 shows the results.

Further, 10 parts by mass of nitrile rubber (Nipol (registered trademark) DN003 manufactured by Zeon Corporation) was dissolved in 90 parts by mass of isophorone to prepare an NBR solution. In 100 parts by mass of the obtained NBR solution were blended 55 parts by mass of silver particles ("agglomerate silver powder G35" manufactured by DOWA Electronics Materials Co., Ltd., average particle size: 5.9 μm), and the mixture was kneaded by a triple roll mill to give a conductive paste. The obtained conductive paste was applied onto a release sheet and dried by a hot air dry oven set to 120°C for 30 minutes or more, to produce a release sheet-attached conductive layer. The specific heat, the heat capacity, and the basis weight of the conductive layer obtained by peeling the release sheet were measured under the above conditions. Table 1 shows the results.

(Electrodes and wiring)

A polyurethane hot-melt sheet was attached to the conductive layer-side surface of the release sheet-attached conductive layer and stacked using a hot pressing machine under the conditions of a pressure of 0.5 kgf/cm$^2$, a temperature of 130°C, and a pressing time of 20 seconds, and the release sheet was peeled to give a polyurethane hot-melt sheet-attached sheet-shaped conductive layer (length: 12 cm and width: 2 cm).

Separately, a polyurethane hot-melt sheet having a length of 13 cm and a width of 2.4 cm was prepared, on which the polyurethane hot-melt sheet-attached sheet-shaped conductive layer (length: 12 cm and width: 2 cm) was stacked, with the polyurethane hot-melt sheet side of the conductive layer directed to the polyurethane hot-melt sheet and one ends in the length direction of the sheet and the conductive layer aligned. These polyurethane hot-melt sheets correspond to the first insulating layer.

Next, a second insulating layer was formed on the conductive layer by stacking the same polyurethane hot-melt sheet as the first insulating layer, so as to cover a part of the first insulating layer and the conductive layer, in a region with a length of 5 cm and a width of 2.4 cm, and from a part 2 cm away from the end. That is, a stretchable electrode part was produced in which an electrode (device connection portion), a wiring portion, and an electrode (detection portion) were longitudinally disposed in this order, the electrode (device connection portion) exposing the conductive layer on the one-end side of the stretchable electrode part and having a size of length 2 cm × width 2 cm, the wiring portion having a stack structure of the first insulating layer/the conductive layer/the second insulating layer, and the electrode (detection portion) exposing the conductive layer on the other-end side and having a size of length 5 cm × width 2 cm. The obtained stretchable electrode part was attached to the warp knitted fabric, with the first insulating layer side directed to the skin-side surface of the warp knitted fabric.

Next, two stretchable electrode parts were symmetrically attached at prescribed positions on the skin-side surface of a T-shirt formed of the warp knitted fabric in each of Examples 1-3 and Comparative Examples 1 and 2, to give a T-shirt having the electrodes and the wiring formed thereon. Further, an electronic unit (My Beat WHS-2) manufactured by UNION TOOL CO. was attached to the surface opposite from the skin-side surface of the T-shirt to produce an electronic unit-attached T-shirt. The number of electrodes provided on the clothing fabric of the front body was two, the total area of the electrode surfaces of the two electrodes for detection was 20 cm$^2$, and the average thickness of the electrodes was 90 μm. The electronic unit-attached T shirts obtained respectively were worn by a subject and evaluated for wear discomfort.

The electronic unit-attached T-shirts produced using the warp knitted fabrics in Examples 1 to 3 had a small difference in the cool feeling between the conductive layer and the warp knitted fabric, and the wear discomfort of the T-shirts was not recognized.

On the other hand, the electronic unit-attached T-shirt produced using the warp knitted fabric in Comparative Example 1 had a high cool feeling of the conductive layer when worn and thus generated the difference in the cool feeling between the conductive layer and the warp knitted fabric, and the wear discomfort of the T-shirt was therefore recognized.

Further, the electronic unit-attached T-shirt produced using the warp knitted fabric in Comparative Example 2 had a high cool feeling of the warp knitted fabric and thus generated the difference in the cool feeling between the conductive layer and the warp knitted fabric, and the wear discomfort of the T-shirt was therefore recognized.

[Table 1]

| | | Clothing fabric (Warp knitted fabric) | | | | | | | Conductive layer | | | | | Formula (1) (Heat capacity A)-(Heat capacity B) | Wear discomfort |
| | Stitch | Non-elastic yarn | | | Elastic yarn | Basis weight (g/m²) | Specific heat (J/g · K) | Heat capacity B (J/K) | Resin NBR (mass%) | Silver particles (mass%) | Basis weight (g/m²) | Specific heat (J/g · K) | Heat capacity A (J/K) | | |
| | | High molecular weight polyethylene (55T/43 f) (mass%) | Polyethylene terephthalate (56T/24f) (mass%) | Nylon 6 (44T/40f) (mass%) | Spandex (78T) (mass%) | | | | | | | | | | |
| Example 1 | Tricot (32Gauge) | 10 | - | 54 | 36 | 213 | 1.13 | 241 | 15 | 85 | 400 | 0.72 | 288 | 47 | NO |
| Example 2 | Tricot (28Gauge) | 10 | - | 67 | 23 | 193 | 1,43 | 276 | 15 | 85 | 400 | 0.72 | 288 | 12 | NO |
| Example3 | Tricot (32Gauge) | 10 | 73 | - | 17 | 197 | 1.50 | 296 | 15 | 85 | 400 | 0.72 | 288 | -8 | NO |
| Comparative Example 1 | Tricot (32Gauge) | - | - | 64 | 36 | 237 | 0,94 | 223 | 15 | 85 | 400 | 0.72 | 288 | 65 | Yes |
| Comparative Example 2 | Tricot (32Gauge) | 20 | 63 | - | 17 | 201 | 1.50 | 302 | 15 | 85 | 400 | 0.72 | 288 | -14 | Yes |

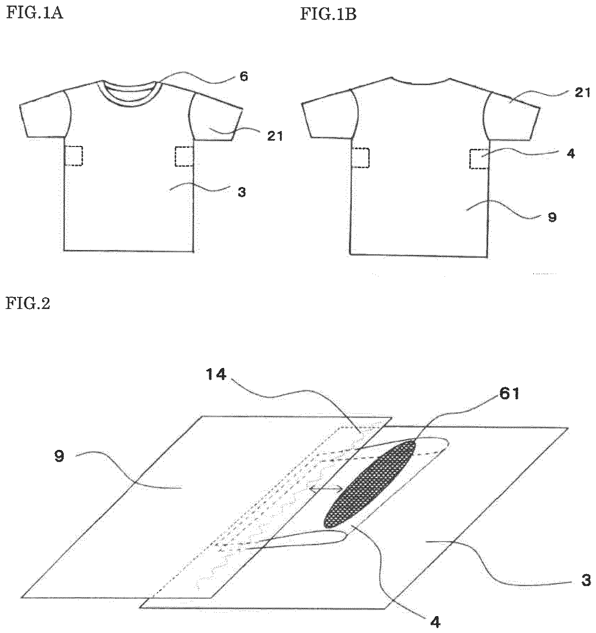

Further, a tab-attached T-shirt shown in Fig. 1A and Fig.1B was produced using the warp knitted fabric obtained in Example 1. Fig. 1A shows a front view of the tab-attached T-shirt, and Fig. 1B shows a back view of the tab-attached T-shirt. At an electrode supporting portion 4 in Fig. 1A and Fig. 1B, an electrode portion 61 shown in Fig. 2 is provided and produced by the following procedures.

On the skin-side surface of the warp knitted fabric (100 mm × 42mm) obtained in Example 1, an electrode was formed so as to have an ellipse shape (minor axis 22 mm × major axis 32 mm) using the conductive layer and the insulating layer that have the same composition as in Example 1, and thus a clothing fabric to which the electrode portion 61 was attached was formed. Next, the clothing fabric to which the electrode portion 61 was attached was sewn into a stitched portion 14 of a front body 3 and a back body 9 as shown in Fig. 2 to form a double-open bag shape, and thus the electrode supporting portion 4 was formed. The shortest distance between the stitched portion 14 and an end of the electrode portion 61 was set to 5 mm. Further, a portion from the electrode portion 61 to the stitched portion 14 was embroidered in a zigzag manner with silver-coated paper that served as wiring, and silver-coated yarn wiring was drawn to a posterior neck portion, overlapping the embroidery wiring and making use of stitched seams, i.e., from the stitched seam on a side portion between the front body 3 and the back body 9 through the stitched seam between a sleeve and the back body 9 to the stitched seam in a shoulder portion between the front body 3 and the back body 9. Thereafter, a connection snap was formed in the posterior neck portion to be connected to the wiring and a detachable electronic unit was connected to the snap to give a tab-attached T-shirt as the biological information measurement garment. The obtained tab-attached T-shirt as the biological information measurement garment was worn by a subject and evaluated for wear discomfort. In spite of the fact that the areas under the armpits, where the electrodes were positioned, were sensitive sites to the cool feeling, the discomfort accompanying the difference in the cool feeling between the electrode and the clothing fabric was not generated.

DESCRIPTION OF REFERENCE SIGNS

| | |
|---|---|
| 3 | front body |
| 4 | electrode supporting portion |
| 6 | neckline |
| 9 | back body |
| 14 | stitched portion |
| 21 | sleeve portion |
| 61 | electrode portion |

**Claims**

1. A garment comprising:

   a clothing fabric; and
   an electrode formed on a skin-side surface of the clothing fabric,
   the clothing fabric having, in the skin-side surface thereof, an area of 30 cm$^2$ or more that is not covered with the electrode and is exposed,
   the electrode including a conductive member, and
   a heat capacity A (J/K) of the conductive member and a heat capacity B (J/K) of the clothing fabric satisfying a following formula (1).

$$-12 \ (J/K) \leq A - B \leq 60 \ (J/K) \ (1)$$

2. The garment according to claim 1, wherein
   the clothing fabric has a heat capacity B (J/K) of 230 (J/K) or more and 300 (J/K) or less.

3. The garment according to claim 1 or 2, wherein
   the conductive member has a heat capacity A (J/K) of 250 (J/K) or more and 350 (J/K) or less.

4. The garment according to any one of claims 1 to 3, wherein
   the clothing fabric has a basis weight of 230 g/m$^2$ or less.

**5.** The garment according to any one of claims 1 to 4, wherein
the clothing fabric includes elastic yarns and non-elastic yarns.

**6.** The garment according to claim 5, wherein
each of the non-elastic yarns includes a polyethylene multifilament yarn.

**7.** The garment according to claim 6, having a blending proportion of the polyethylene multifilament yarn in 100 mass%
of the clothing fabric of 2 mass% or more and 18 mass% or less.

**8.** The garment according to any one of claims 1 to 7, wherein
the conductive member is a conductive layer and contains 20 mass% or more and 98 mass% or less of a conductive
filler.

**9.** The garment according to any one of claims 1 to 8, wherein
the conductive member is formed of a conductive fabric, and when the conductive fabric has a load of 14.7 N applied
thereto in a body length direction or a body width direction, the conductive fabric has an extension percentage of
3% or more and 60% or less in at least one of the directions.

**10.** The garment according to any one of claims 1 to 9, being an underwear top.

**11.** The garment according to any one of claims 1 to 9, being an underwear bottom.

**12.** The garment according to any one of claims 1 to 9, being a band-shaped fabric.

FIG.1A

FIG.1B

FIG.2

## INTERNATIONAL SEARCH REPORT

| International application No. |
|---|
| PCT/JP2020/014441 |

**A. CLASSIFICATION OF SUBJECT MATTER**
A61B 5/0408(2006.01)i; A41B 9/06(2006.01)i; A41D 13/12(2006.01)i; A41D 31/04(2019.01)i
FI:   A61B5/04 300M; A61B5/04 300W; A41D13/12 145; A41D13/12 154; A41B9/06 Z; A41D31/04 Z; A41D13/12 181
According to International Patent Classification (IPC) or to both national classification and IPC

**B. FIELDS SEARCHED**

Minimum documentation searched (classification system followed by classification symbols)
A61B5/04-5/0488; A41B9/06; A41D13/12; A41D31/04

| Documentation searched other than minimum documentation to the extent that such documents are included in the fields searched | |
|---|---|
| Published examined utility model applications of Japan | 1922–1996 |
| Published unexamined utility model applications of Japan | 1971–2020 |
| Registered utility model specifications of Japan | 1996–2020 |
| Published registered utility model applications of Japan | 1994–2020 |

Electronic data base consulted during the international search (name of data base and, where practicable, search terms used)

JSTPlus/JMEDPlus/JST7580 (JDreamIII); PubMed; IEEE Xplore; 医中誌 WEB (Ichushi WEB)

**C. DOCUMENTS CONSIDERED TO BE RELEVANT**

| Category* | Citation of document, with indication, where appropriate, of the relevant passages | Relevant to claim No. |
|---|---|---|
| A | JP 2017-35457 A (TOYOBO CO., LTD.) 16.02.2017 (2017-02-16) paragraphs [0002]-[0003], [0009]-[0011] | 1–12 |
| A | WO 2015/115441 A1 (NIPPON TELEGRAPH AND TELEPHONE CORP.) 06.08.2015 (2015-08-06) paragraphs [0006]-[0036], fig. 1 | 1–12 |

☐ Further documents are listed in the continuation of Box C.   ☒ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
|---|---|
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 04 June 2020 (04.06.2020) | 16 June 2020 (16.06.2020) |

| Name and mailing address of the ISA/ | Authorized officer |
|---|---|
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

| INTERNATIONAL SEARCH REPORT Information on patent family members | | International application No. PCT/JP2020/014441 | |
|---|---|---|---|
| Patent Documents referred in the Report | Publication Date | Patent Family | Publication Date |
| JP 2017-35457 A | 16 Feb. 2017 | (Family: none) | |
| WO 2015/115441 A1 | 06 Aug. 2015 | US 2016/0374615 A1 paragraphs [0005]-[0035], fig. 1 EP 3100677 A1 CA 2938025 A1 KR 10-2016-0113603 A CN 105939660 A TW 201538131 A CL 2016001855 A | |

Form PCT/ISA/210 (patent family annex) (January 2015)

**EP 3 949 847 A1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2017029692 A
- JP 2019067965 A